# EUROPEAN PATENT APPLICATION

(11) **EP 3 029 136 A1**
(43) Date of publication of application: **08.06.2016**
(21) Application number: 14822246.6
(22) Date of filing: 08.07.2014
(51) Int. Cl.: C12N 1/12, C12M 1/00, C12M 1/04, C12M 1/42, C10L 1/02

(54) **INSTALLATION FOR OBTAINING BIOMASS BY MEANS OF THE CULTURE OF ALGAE AND OBTAINING A BIOREFINED PRODUCT FOR THE PRODUCTION OF BIO-OIL AND BIOPRODUCTS AND A METHOD FOR OBTAINING SAME**

(30) Priority: 09.07.2013 ES 201300646
(71) Applicant: Dawn Demmier, Viviene, 03110 Muchamiel (Alicante) (ES); Martin de la Iglesia, Sonia, 03110 Muchamiel (Alicante) (ES)
(72) Inventor: DAWN DEMMER,, Vivienne, E-03110 Muchamiel (Alicante) (ES); MARTIN DE LA IGLESIA,, Sonia, E-03110 Muchamiel (Alicante) (ES)
(74) Representative: Carvajal y Urquijo, Isabel
(86) International application number: PCT/ES2014/070559
(87) International publication number: WO 2015/004300

(57) **Abstract**

The present invention describes an installation for obtaining biomass by means of the culture of algae and obtaining a biorefined product for the production of bio-oil and bioproducts and the method for obtaining same by means of a closed axenic process in photounits. In the installation a culture medium is recreated that is similar to that which may be encountered in natural marine waters where the microorganisms develop and which is based on the use of seawater or brine, natural or artificial solar radiation, vitamins, nutrients, a stationary CO₂ supply and on recirculation of both CO₂ and water produced in all intermediate steps of the process. The production method allows the possibility, in the installation, for the provision of divisions of the photounits such that different classes of single-cell microalgae can be cultured therein, i.e., in the phototubes making up said units there is only one type of single-cell alga. Extractions are performed in cycles of 12 to 16 hour from the first biomass extraction therefore the production of bionutrient and bioenergetic products occurs continuously and a 95% biomass production yield is obtained.

## Description

### Object of the Invention

As expressed in the title of the present specification, the invention relates to an industrial installation for obtaining nutritive and energetic products and the method for obtaining these products from the culture of algae using greenhouse gases, particularly CO₂, as nutrients.

The field of application of the present invention within the field of biotechnology is the phytotechnology industry implementing solutions to scientific problems and the field of engineering based on plants such as algae or others, and it comprises the development of clean technologies such as capturing harmful or greenhouse gases and transforming same into nutrients or energetic products.

### Background of the Invention

Biotechnology arises around approximately 6000 B.C. when Neolithic cultures start to make use of grape fermentation to produce wine or the Babylonians use yeasts of microbial origin to perform same with beer. Today, industrial biotechnology involves the microbiological production of enzymes acting as biocatalysts facilitating and accelerating complex biochemical reactions that are essential for many daily applications and turning it into a new and powerful technology used in an increasingly wide range of applications.

Today, it is necessary to prevent the scarcity of natural resources such as water or oxygen and to make development compatible with energy, the environment and nutrition. In addition to the concern caused by the energy and nutritional needs of the earth population, there is also a growing concern with respect to the reduction in the thickness of ozone layer as a result of the excessive emissions of greenhouse gases including, among others, CO₂ and CH₄, and the distribution of potable water affected by the increase in earth temperature and by contamination derived not only from the aforementioned gases but also from the massive use of other products such as fertilizers, herbicides, pesticides, fungicides, etc.

All these factors force the research for alternative solutions independent from the climate conditions and/or problems derived from the location of natural resources.

Diatoms, bacteria or euglenoids are different groups of algae that do not compete with food crops. For reproduction, these algae only require sunlight, nutrients, CO₂ and seawater, and a technological culture system capable of imitating their natural growth environment.

In this field, most of the microorganism culture plant designs focus on the production of biofuels based on capturing CO₂ emissions and converting gases into energy of the biomass by means of the photosynthesis. Closed vertical phototubes are not used for biofuel production, most of these culture systems being developed in open tanks more suitable for this purpose. However, these processes adapted for obtaining biofuels do not contain the necessary concentration and purity for obtaining a wide range of products with high added value.

The culturing process proposed by the present invention consists of a succession of steps of biomass production established in accordance with the collection, separation and extraction phases as well as the reintroduction of waste in the water and gas lines: all the discharges are recycled.

The developed plant is based on an innovative process of axenic culture and single-cell algae processing in aqueous medium taking place inside a photounit in which there is injected a gas stream with a high CO₂ content and marine water enriched with nutrients.

The harvested algae are compressed and accumulated, said culture going through a series of successive treatment steps from which biomass with a controlled moisture content is obtained. Lipids, fatty acids, amino acids and proteins are extracted from this biomass. In each separation operation, the so-called "waste" made up of the remaining nutrients, biomass and water which are physicochemically processed and reintroduced in the process as raw materials is obtained.

In order to achieve an efficient process from the energy viewpoint, preventing the emission of gaseous or liquid contaminants, the process is developed according to the aforementioned phases to prevent said emissions. This process is balanced energy wise, i.e., it does not use more energy than that produced and, for this reason, the development uses other renewable energies to compensate for said energy consumption.

The main application focuses on the transformation of greenhouse gases, particularly CO₂, by means of the biomass from an intensive culture of certain species of algae. Various straining and draining techniques are used within a closed culture system, using transparent vertical phototubes. By means of this process, it is possible to obtain basic products such as fats and proteins that can be incorporated for human/animal consumption before finally obtaining biofuels.

Finally, combination of the production process with renewable electricity and heat generating technologies results in an almost zero energy balance of the assembly and allows the adaptation thereof to criteria of maximum efficiency at all time.

In other conventional processes, the production of biofuels and products with added value based on the culture of algae requires great electricity and heat consumptions significantly increasing the production costs, however, the process proposed by the present invention, incorporating renewable geothermal technology significantly reduces external energy needs, likewise, the costs derived from its installation are compensated for by means of selling the excess electricity to power grids and CO₂ emission rights and the byproducts obtained from the residues as biochemical or bioclastic compounds, etc.

As the most noteworthy results, for an average plant in an ideal location with a conservative estimated production of 0.8 g/day for 330 days:
1. The production costs are ± 14.41 €/Kg of dry biomass compared with 35 €/Kg of a standard process
2. The production is 276 ton/ha of dry biomass compared with 100 ton/ha of a standard process.
3. The implementation cost is less than 4,500 €/m³ of culture with respect to 10,000 €/m³ of a standard process.
4. As a result, the energy costs of the process are optimized to less than 5 kW/Kg of dry mass.

### Description

The installation and the method for obtaining biomass is integrated within various fields of biotechnology and is included as a result of its technical process within the field of ecotechnology, in which knowledge of processes typical of the ecosystems and societies are combined with a technological application using biological systems, living organisms such as microalgae for producing biomass which is then biorefined for the purpose of obtaining valuable nutritive products and oil that can be used in different commercial applications such as pharmaceutical products, cosmetics, food supplements and other market segments, including biofuels.

Within the biotechnology and biorefinery industry for the production of products with high added value and biofuels by means of continuous production systems using phytoplankton from which energy is obtained by means of the process of photosynthesis, the microorganisms used must therefore live on well illuminated surface layers of oceans, seas or other water masses. However, the ecological and biodiversed process herein described uses other renewable energies, non-potable water and non-agricultural lands, and recycles captured industrial gas emissions, mainly CO₂.

The sequence of the process and the prior preparation determine the results of the final byproducts and the object of this invention is to describe the necessary production line, from culturing algae to biorefining the biomass and the bio-oil.

The purpose of the installation or processing plant, object of the present specification, is to produce nutritive and energetic biotechnological products based on using cultures of algae that capture atmospheric CO₂ after performing different intermediate separations in accordance with the following diagram:

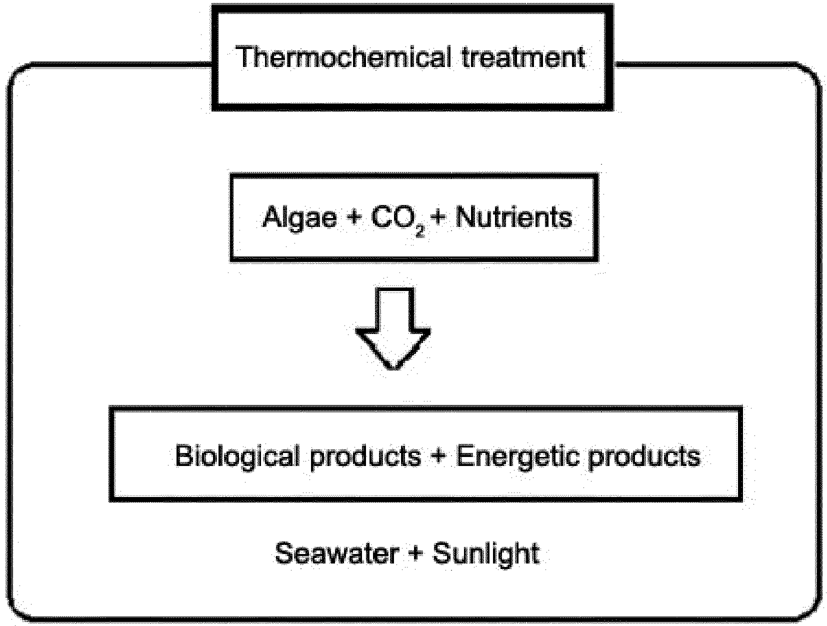

An exhaustive conversion of harmful gases by means of a superintensive alga culture system using seawater, sunlight and other nutrients such as phosphates and nitrogen as main inputs takes place. The method is developed according to a closed circuit in which all the intermediate products from each of the steps are used. The final products can be intended both for human/animal nutrition and for industrial use in order to obtain energy based on the consideration thereof as biofuels.

The objectives of the method described in the present specification are:
- To achieve a dense biomass production starting in the intensive microalga culture phase allowing extractions every 12-16 hours which includes efficiently capturing CO₂ and converting it into energetic and nutritive products and recycling excess CO₂ by recirculating it again to the culture medium, as well as water and nutrients contained therein.
- To set forth that two main culture types can be combined to produce two types of final products. One valid only for nutritive products with added value and another to produce only biofuels. Furthermore, both systems can also be arranged under an enclosure protecting the culture method from elements of nature, such as ice, cold, wind and snow and from bacteria.

When the culture method is capable of improving the acceleration of alga growth, CO₂ consumption within the system will be high and the complete process can be considered efficient in capturing CO₂.

From the culture to the biomass biorefining process, large amounts of energy in the form of electricity are required, therefore incorporating renewable technologies so that the process can have reduced costs and be considered energetically balanced is decisive.

Among the products obtained by means of the application of the method that has been described, the following must be highlighted in a non-limiting manner:

| Types of product | |
|---|---|
| Bio-oil | Biodiesel, naphthas, biopetroleum |
| Fibers | Animal feed |
| For agricultural use | Fertilizers |
| Vitamins | B12, beta-carotenes |
| Acids | Omega-3, 6 and 9 |
| Amino acids | Lysine, valine, leucine |
| Minerals | Iodine, sodium |

The following chart specifically shows the products obtained by means of the culture of algae in the present method and the industrial application thereof

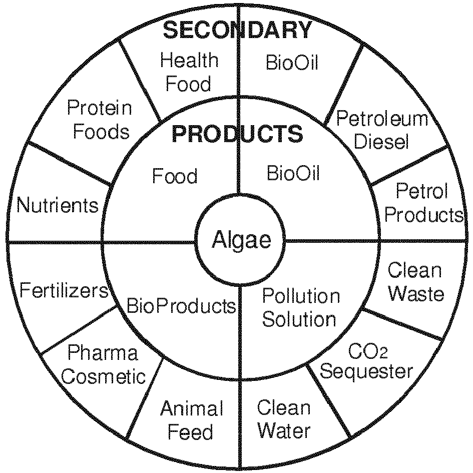

To achieve the objectives described above which mainly in economic commercial terms can be defined as obtaining greater amount of biomass with extractions performed every 12-16 hours and a high quality due to the culture medium and to the refining process thereof, the following must be highlighted:
- For the axenic culture of microalgae fungicides and antibiotics are not used, giving the obtained products a better quality than those obtained from conventional processes;
- By means of divisions of the photounits, it is possible to use different species within one and the same production plant, for example, a species rich in lipids and another species low in lipids, such that the final production of both complement one another to give rise to a fuel rich in hydrocarbons.
- All the waste in each step, even the gases, are treated and reintroduced in the culture method of the plant and reused;
- The design of the injection of liquid and gas flows
- Before the introduction thereof into the photounits, optionally CO₂ from the mixed gas stream can be separated from other substances, such as SOₓ, NOₓ and ash by means of washing and compression processes. This adjustment of the gas stream is performed by cooling the gases before injection.
- The uniform irradiation of the growth system depending on the algae used and on the required production results, determines the quality and the amount of carbohydrates, proteins, sugars and fatty acids.

The installation for carrying out the production method can be designed such that its operation is adapted to specific weather conditions, obtaining the greatest efficiency possible. Irradiation differs throughout the year and more is required in some areas than in others, such that the path of the culture is adapted from its initial culture to its extraction, and is likewise adapted between extractions. There are many different possibilities for producing bioproducts using microalgae, but optimization of the complete process is vital, as mentioned in "Microalgal Photobioreactors: Scale-up and Optimization", September 12, 2003, by Prof. Dr. A. Richmond, Prof. Dr. Tredici, Prof. Dr. Ir. WH Rulkens and Prof. Dr. L.R. Mur, and according to the principles of Mr. Clemens Posten of the Institute of Life Science Engineering. Division of Bioprocess Engineering, University of Karlsruhe, Germany, May 29, 2009.

This process uses, but is not limited to, autotrophic species such as *Nannochloropsis oculata, Nannochloropsis salina, Nannochloropsis oceanica, Dunaliella salina* and *Chaetocerotaceae.* There are two types of systems using these cultures of algae: the first system in open channels or tanks in which a direct exchange with the atmosphere between the culture and the circulating air occurs, and another system in which the culture is enclosed in plastic bags, polycarbonate tubes or closed tanks, allowing the passage of solar radiation but not the material exchange.

All the parameters such as solar radiation, pH, salinity, nutrients, conductivity, temperature, density, etc., and all the mechanisms of operation are controlled by a specific control software program, optimizing the efficiency and versatility of the production plant.

By means of incorporating elements such as electric pumps, pipes, filling processes, photounits, waste chambers and different containers, with basic UV rays, active carbon filters and/or ozonation contamination and infection is prevented, the cleaning and disinfection thereof is made easier and the overall process efficiency is improved.

The entire culturing process takes place in sealed (closed) photounits in continuous production. The culture system is based on solar radiation, a stationary CO₂ supply, nutrients and a seawater/brine-based medium.

Success in the culture of phytoplankton/algae also depends on the capacity of the system to imitate the natural conditions of the medium in which they usually grow, therefore CO₂, temperature, turbidity, pH, and salinity of the mixture are continuously monitored and the subsequent movements of the medium for replicating ocean currents and tides, such that a medium that is similar to that which may be encountered in natural waters is recreated.

The culturing process can be defined as "electrically charged photounits" in which an "electric charge" is subsequently introduced to produce more electrons that are required by the process of photosynthesis.

The culturing process does not use antibiotics and/or fungicides at all.

Up until now no other process in the field of biotechnology has combined renewable energies such as wind energy, geothermal energy and solar energy for the entire plant or in waste treatment plants to produce the necessary heat and energy and at the same time clean contamination and CO₂ emissions, and the entire process being covered by greenhouses which can also help in the artificial lighting of the process, as well as capturing rainwater/condensation that can then be used in the processing plant.

Throughout the process, all the discharges such as the CO₂ released during the culture are recaptured in the upper part of the photounits and recycled through CO₂ supply to said phototubes. The culture water is recycled throughout the process from the culture until the separation, centrifugation and finally at the bioproduct and biofuel extraction point.

In colder areas and/or in areas with less solar radiation, structures such as greenhouses are incorporated to protect the culturing process. The greenhouse, however, does not only serve as protection against cold, but can also be used for capturing rain/moisture, and filtering and cleaning same for human consumption or for being used in the plant itself.

This installation and its method for obtaining biomass can also be fully operative in cold or frozen areas as a result of incorporating other renewable energies. Furthermore, the process can work in the absence of sunlight, tubes with artificial lighting being able to be used arranged inside the structure of the greenhouse and located freely on the ground in some areas.

The main differentiating features with respect to the standard process can be summarized as:
- The operation is performed in a closed cycle, reusing the waste from each of the steps;
- The absence of intermediate steps leads to better harvesting methods with respect to the existing technologies;
- The thermochemical treatment equipment
- The plant control and photounit differentiation allow complete adaptability to the environmental and alga growth conditions;

### Description of the Process Diagram

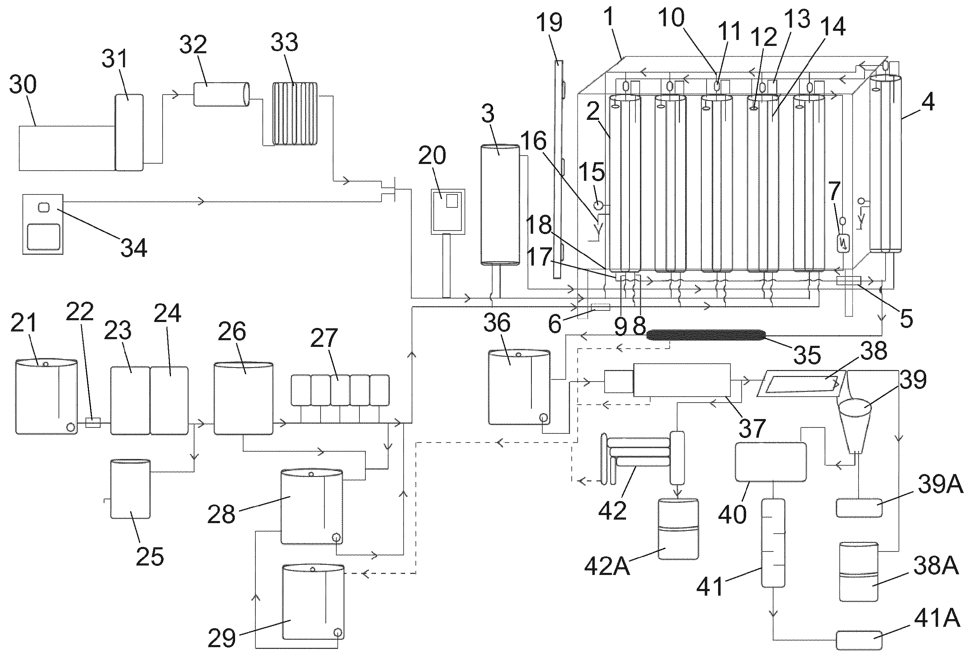

To complement the description that is being made of the installation and the method for obtaining biomass and biorefined product and in order to provide better understanding the features of the invention, an illustrative and non-limiting process diagram which will be described in the embodiment of the invention is attached as Figure 1 to the present specification as an integral part thereof:

### Preferred Embodiment of the Invention

### Prior preparation step

A stream of seawater or brine enriched with nutrients and vitamins which has in turn been previously microfiltered and disinfected is first injected.

The following components of the installation are used in this phase:
- 1 seawater accumulation tank (21) from which said seawater is used in the culturing process;
- 1 automatic electric pump (22) for circulating water from the accumulation to the next phase;
- 1 desalinating unit (23) for reducing the salt content of water;
- 1 water disinfecting unit (24) for eliminating any chemical product or material that may be damaging to the culture;
- 1 storage tank (25) for the excess clean water;
- 1 ultraviolet chamber (26) for the treatment of any bacterium that may still be present in the water;
- 5 nutrient supply tanks (27) for covering the nutritional needs of the culture;
- 1 mixing tank (28) in which the treated water and the nutrients are mixed, such that supply to the photounits is uniformly distributed;
- 1 water return tank (29) for treating all the return water from the entire process.

Likewise, a stream of gas which contains CO₂ and has been filtered in accordance with the specific needs of the process is introduced into the photounits.

The following components of the installation are used to carry out this phase of the process:
- 1 gas capturing unit (30) for capturing gases which can be obtained from industrial emissions;
- 1 CO₂ filtration unit (31). It separates CO₂ from NOx and SOx present in the gases. CO₂ and N₂ are sent to the culture photounits and the remaining gases are neutralized;
- 1 prepared CO₂ mixing tank (32);
- 1 CO₂ pressure regulator and cooler (33) for cooling the CO₂ before entering the culturing process;
- 1 air compressor (34).

Finally, in this step a filtering of the selected algae is introduced into the photounits once the optimal sunlight conditions which allow carrying out the process of photosynthesis are assured.

### Culturing step:

Depending on the characteristics of the nutrients and vitamins used for feeding the microalgae, the product is determined in the final extraction. This system does not use any type of fungicides or antibiotics.

The culturing process is completely axenic in each photounit and uses as microorganisms autotrophic single-cell algae but is not limited to species such as *Nannochloropsis oculata, Nannochloropsis salina, Nannochloropsis oceanica, Dunaliella salina* and *Chaetocerotaceae,* heterotrophic algae or mixture thereof.

The process whereby culturing is carried out takes place inside the phototubes and these can be:
.- External phototubes which are transparent, made of PVC or polycarbonate and arranged vertically inside the photounits. The size thereof can vary between 1 and 10 meters. Generally, they are circular shaped, although in some cases they can be square. The circular tubes have a diameter varying between 100 and 400 mm.
.- Internal phototubes which do not necessarily have to be transparent and are made of PVC or polycarbonate. Despite the absence of sunlight, the culture can develop by means of using tubes with artificial lighting arranged inside greenhouses or freely on the ground.

Each photounit contains no less than 5 and up to 30 phototubes and they are spaced from one another no less than 1 cm and up to 60 cm in order to allow natural entry of sunlight. The phototubes making up each photounit are arranged parallel to other photounits at a distance between 2 and 5 meters between each row.

The phototubes imitate the course of nature within the field of photosynthesis and make capturing of CO₂ by the microorganisms easier so that they can develop.

In this step of the process, the installation is formed by the following components:
- 1 rectangular structure of the photounit made of steel which is embedded 0.5 meters in a concrete support foundation (1);
- From 5 to 30 phototubes (2) located in each unit arranged parallel to other photounits at a distance of at least 2 meters and up to 5 meters between each row, it must consists of 2 rows;
- 1 supply phototube (3) for each photounit which allows concentrating the culture within the culturing process;
- 1 photocirculator (4) located in the center of each unit and parallel-wise, which allows mixing the culture;
- 1 heat exchanger (5) for every parallel photounit to control the temperature of the culture and keep it constant;
- 1 electric pump (6) for every unit to move the culture;
- 1 electric charging unit (7) for every parallel photounit to favor the flow of electrons through the culture;
- 1 input valve (8) for supplying water and nutrients;
- 1 input valve (9) for supplying CO₂ and air
- 1 pressure release valve (10) to allow discharging oxygen into the atmosphere;
- 1 gas separator (11) to separate the CO₂ and the O₂;
- 1 sensor unit (12) for measuring water level in each phototube;
- 1 sensor unit for measuring pH, salinity, temperature, turbidity, CO₂, oxygen and nutrient input (13);
- 1 hydrometer (14) for measuring the culture which allows the successful extraction thereof from the photounits;
- 1 manometer (15) for controlling pressures inside the phototubes;
- 1 culture draw-off tap per phototube (16);
- 1 network of recirculation pipes (17) for each photounit;
- 1 network of recirculation pipes (18) for each photounit;
- 3 lamps with artificial lighting (19) per photounit to aid in photosynthesis inside the phototubes;
- 1 control panel (20) per photounit arranged in parallel for automatically controlling and monitoring the input and output of the phototubes.

### Biomass extraction step

The first biomass extraction takes place between 8 and 14 days from the first implementation of the method and occurs when the biomass concentration reaches a density varying between 100 and 600 millions of cells/ml. After this first extraction, the following extractions will occur continuously every 12-16 hours.

The yield achieved is at least 95% microalga content.

Once the first biomass extraction from the photounits has been performed the method for obtaining final products consists of:
- 1 first separation (35) in a piece of equipment particularly designed by GSBS for this purpose and which is largely responsible for the high yield obtained. At least 25% of water is successfully separated from the solid fraction in this phase. The separated water is conducted to the return tank (29) for being reintroduced into the process;
- 1 accumulation tank (36) for the culture which will contain the output product of the first separation;
- 1 centrifugation (37) for the subsequent separation of water from the solids of this operation, water is also separated from the solid fraction, this water is conducted to the return tank (29);
- 1 conveyor (38) for conveying the biomass from one phase to the next; this conveyor can also help to reduce the moisture content of the mass;
- 1 collection point (38A) at which the mass can be sold directly as product with a considerable amount of moisture;
- 1 spray drying process (39) for preparing the biomass for product extraction;
- 1 product collection point of the spray separator (39A) at which the dry biomass can be sold directly as final product;
- 1 product extraction process (40) whereby lipids and proteins are extracted;
- 1 fractioning process (41) whereby lipids are subsequently processed for obtaining products with high added value, antioxidants and nutritive products;
- 1 collection point for processed products (41 A);
- 1 liquefaction process (42) as an alternative option for the mass which can use the biomass with high water content to produce bio-oil and to subsequently process it into biofuel. This process contains return of water towards the water return tank (29);
- 1 biofuel collection point (42A) in which said biofuel is introduced into barrels.

## Claims

1. An installation for obtaining biomass by means of the culture of algae and obtaining a biorefined product for the production of bio-oil and bioproducts and method for obtaining same, **characterized in that** the installation is configured with the following components :
• 1 seawater accumulation tank (21)
• 1 automatic electric pump (22)
• 1 desalinating unit (23)
• 1 water disinfecting unit (24)
• 1 storage tank (25)
• 1 ultraviolet chamber (26)
• 5 nutrient supply tanks (27)
• 1 mixing tank (28)
• 1 water return tank (29)
• 1 gas capturing unit (30)
• 1 CO₂ filtration unit (31)
• 1 prepared CO₂ mixing tank (32)
• 1 CO₂ pressure regulator and cooler (33)
• 1 air compressor (34)
• 1 rectangular structure of the photounit made of steel, embedded 0.5 meters in a concrete support foundation (1)
• from 5 to 30 phototubes (2) in each unit,
• 1 supply phototube (3) for every photounit
• 1 photocirculator (4)
• 1 heat exchanger (5) for every photounit
• 1 electric pump (6) for every unit
• 1 electric charging unit (7) for every parallel photounit
• 1 input valve (8)
• 1 CO₂ and air supply input valve (9)
• 1 pressure release valve (10)
• 1 gas separator (11)
• 1 sensor unit (12) for measuring water level
• 1 sensor unit for measuring pH, salinity, temperature, turbidity, CO₂, oxygen and nutrient input (13)
• 1 hydrometer (14)
• 1 manometer (15)
• 1 culture draw-off tap per phototube (16)
• 1 network of recirculation pipes (17) for each photounit
• 1 network of recirculation pipes (18) for each photounit
• 3 lamps with artificial lighting (19) per photounit
• 1 control panel (20) per photounit
• 1 first separating unit (35)
• 1 accumulation tank (36) for the culture
• 1 centrifugation unit (37)
• 1 conveyor unit (38)
• 1 collection point (38A)
• 1 spray drying unit (39)
• 1 product collection point of the spray separator (39A)
• 1 product extraction unit (40)
• 1 fractioning unit (41)
• 1 collection point for processed products (41 A)
• 1 liquefaction unit (42)
• 1 biofuel collection point (42A).

2. The installation for obtaining biomass by means of the culture of algae and obtaining a biorefined product for the production of bio-oil and bioproducts and method for obtaining same, **characterized in that** the method is configured under the following steps :
.- Prior preparation
.- Culturing
.- Biomass extraction for obtaining bionutrient and bioenergetic products
Prior preparation step
A stream of seawater or brine enriched with nutrients and vitamins which has in turn been previously microfiltered and disinfected is first injected.
The following components of the installation are used in this phase:
• 1 seawater accumulation tank (21) from which said seawater is used in the culturing process;
• 1 automatic electric pump (22) for circulating water from the accumulation phase to the next phase;
• 1 desalinating unit (23) for reducing the salt content of water;
• 1 water disinfecting unit (24) for eliminating any chemical product or material that may be damaging to the culture;
• 1 storage tank (25) for the excess clean water;
• 1 ultraviolet chamber (26) for the treatment of any bacterium that may still be present in the water;
• 5 nutrient supply tanks (27) for covering the nutritional needs of the culture;
• 1 mixing tank (28) in which the treated water and the nutrients are mixed, such that supply to the photounits is uniformly distributed;
• 1 water return tank (29) for treating all the return water from the entire process;
Likewise, a stream of gas which contains CO₂ and has been filtered in accordance with the specific needs of the process is introduced into the photounits.
The following components of the installation are used to carry out this phase of the process:
• 1 gas capturing unit (30) for capturing gases which can be obtained from industrial emissions;
• 1 CO₂ filtration unit (31). It separates CO₂ from NOx and SOx present in the gases. CO₂ and N₂ are sent to the culture photounits and the remaining gases are neutralized;
• 1 prepared CO₂ mixing tank (32);
• 1 CO₂ pressure regulator and cooler (33) for cooling the CO₂ before entering the culturing process;
• 1 air compressor (34);
Finally, in this step a filtering of the selected algae is introduced into the photounits once the optimal sunlight conditions which allow carrying out the process of photosynthesis are assured.
Culturing step:
Depending on the characteristics of the nutrients and vitamins used for feeding the microalgae, the product is determined in the final extraction. This system does not use any type of fungicides or antibiotics.
The culturing process is completely axenic in each photounit and uses as microorganisms autotrophic single-cell algae but is not limited to species such as *Nannochloropsis oculata, Nannochloropsis salina, Nannochloropsis oceanica, Dunaliella salina* and *Chaetocerotaceae,* heterotrophic algae or mixture thereof.
The process whereby culturing is carried out takes place inside the phototubes and these can be:
.- External phototubes which are transparent, made of PVC or polycarbonate and arranged vertically inside the photounits. The size thereof can vary between 1 and 10 meters. Generally, they are circular shaped, although in some cases they can be square. The circular shapes have a diameter varying between 100 and 400 mm.
.- Internal phototubes which do not necessarily have to be transparent and are made of PVC or polycarbonate. Despite the absence of sunlight, the culture can develop by means of using tubes with artificial lighting arranged inside greenhouses or freely on the ground.
Each photounit contains no less than 5 and up to 30 phototubes and they are spaced from one another no less than 1 cm and up to 60 cm in order to allow natural entry of sunlight. The phototubes making up each photounit are arranged parallel to other photounits at a distance between 2 and 5 meters between each row.
The phototubes imitate the course of nature within the field of photosynthesis and make capturing of CO₂ by the microorganisms easier so that they can develop.
In this step of the process, the installation is formed by the following components
• 1 rectangular structure of the photounit made of steel which is embedded 0.5 meters in a concrete support foundation (1);
• from 5 to 30 phototubes (2) located in each unit arranged parallel to other photounits at a distance of at least 2 meters and up to 5 meters between each row, it must consists of 2 rows;
• 1 supply phototube (3) for each photounit which allows concentrating the culture within the culturing process;
• 1 photocirculator (4) located in the center and parallel of each unit which allows mixing the culture;
• 1 heat exchanger (5) for every parallel photounit to control the temperature of the culture and keep it constant;
• 1 electric pump (6) for every unit to move the culture;
• 1 electric charging unit (7) for every parallel photounit to favor the flow of electrons through the culture;
• 1 input valve (8) for supplying water and nutrients;
• 1 input valve (9) for supplying CO₂ and air
• 1 pressure release valve (10) to allow discharging oxygen into the atmosphere;
• 1 gas separator (11) to separate CO₂ and O₂;
• 1 sensor unit (12) for measuring water level in each phototube;
• 1 sensor unit for measuring pH, salinity, temperature, turbidity, CO₂, oxygen and nutrient input (13);
• 1 hydrometer (14) for measuring the culture which allows the successful extraction thereof from the photounits;
• 1 manometer (15) for controlling pressures inside the phototubes;
• 1 culture draw-off tap per phototube (16);
• 1 network of recirculation pipes (17) for each photounit;
• 1 network of recirculation pipes (18) for each photounit;
• 3 lamps with artificial lighting (19) per photounit to aid in photosynthesis inside the phototubes;
• 1 control panel (20) per photounit arranged in parallel for automatically
controlling and monitoring the input and output of the phototubes;
Biomass extraction step
The first biomass extraction takes place between 8 and 14 days from the first implementation of the method and occurs when the biomass concentration reaches a density varying between 100 and 600 millions of cells/ml. After this first extraction, the following extractions will occur continuously every 12-16 hours.
The yield achieved is at least 95% microalga content.

3. The installation for obtaining biomass by means of the culture of algae and obtaining a biorefined product for the production of bio-oil and bioproducts and method which uses it characterized according to the preceding claim in that once the first extraction has been performed the method for obtaining final products consists of:
• 1 first separation (35) in a piece of equipment particularly designed by GSBS for this purpose and which is largely responsible for the high yield obtained. At least 25% of water is successfully separated from the solid fraction in this phase. The separated water is conducted to the return tank (29) for being reintroduced into the process;
• 1 accumulation tank (36) for the culture which will contain the output product of the first separation;
• 1 centrifugation (37) for the subsequent separation of water from the solids of this operation, water is also separated from the solid fraction, this water is conducted to the return tank (29);
• 1 conveyor (38) for conveying the biomass from one phase to the next; this conveyor can also help to reduce the moisture content of the mass;
• 1 collection point (38A) at which the mass can be sold directly as product with a considerable amount of moisture;
• 1 spray drying process (39) for preparing the biomass for product extraction;
• 1 product collection point of the spray separator (39A) at which the biomass can be sold directly as final product;
• 1 product extraction process (40) whereby lipids and proteins are extracted;
• 1 fractioning process (41) whereby lipids are subsequently processed for obtaining products with high added value, antioxidants and nutritive products;
• 1 collection point for processed products (41 A);
• 1 liquefaction process (42) as an alternative option for the mass which can use the biomass with high water content to produce bio-oil and to subsequently process it into biofuel. This process contains return of water towards the water return tank (29);
• 1 biofuel collection point (42A) in which said biofuels is introduced into barrels;

4. The installation for obtaining biomass by means of the culture of algae and obtaining a biorefined product for the production of bio-oil and bioproducts and method for obtaining same characterized according to the preceding claims in that it does not generate residues or produce residual waters. The net balance of CO₂ is nil; all the CO₂ fed to the phototubes, as well as any excess produced therein or not fixed, is recaptured in the upper part of the phototube and reintroduced into the original method for supplying CO₂. Likewise, the waste water is again recycled to the process after each phase.

5. The installation for obtaining biomass by means of the culture of algae and obtaining a biorefined product for the production of bio-oil and bioproducts and method for obtaining same characterized according to the preceding claims in that it uses renewable energy sources for supplying electricity and heat and the components of the installation, the production method and the products obtained are controlled by means of a suitable software program.
